# EUROPEAN PATENT SPECIFICATION

(11) **EP 1 272 179 B1**
(45) Date of publication and mention of the grant of the patent: **02.12.2009**
(21) Application number: 01929462.8
(22) Date of filing: 28.03.2001
(51) Int. Cl.: A61K 31/403, A61K 9/14, A61P 9/00

(54) **HYDROPHILIC MOLECULAR DISPERSE SOLUTIONS OF CARVEDILOL**
HYDROPHILE MOLEKULARDISPERSE LÖSUNGEN VON CARVEDILOL
SOLUTIONS DISPERSEES MOLECULAIRES HYDROPHILES DE CARVEDILOL

(30) Priority: 03.04.2000 EP 00107093
(43) Date of publication of application: 08.01.2003
(73) Proprietor: F. Hoffmann-La Roche AG, 4070 Basel (CH)
(72) Inventor: GABEL, Rolf-Dieter, 68723 Schwetzingen (DE); WIRL, Alexander, 67259 Heuchelheim (DE); PREIS, Walter, 67433 Neustadt (DE); NEUGEBAUER, Guenter, 68309 Mannheim (DE)
(74) Representative: Brodbeck, Michel
(86) International application number: PCT/EP2001/003502
(87) International publication number: WO 2001/074357

(56) References cited:
- WO-A-93/23022
- DE-A- 19 809 242
- DE-A- 19 816 036

## Description

The present invention is concerned with concentrated solid or semi-solid, hydrophilic molecular dispersed solutions of carvedilol and/or of a pharmaceutically acceptable salt thereof, pharmaceutical administration forms comprising such solutions as well as their use for the manufacture of a medicament for the treatment or prophylaxis of illnesses.

Carvedilol is a non-selective β-blocker with a vasodilating component, which is brought about by antagonism to the α₁-adrenoreceptors. Moreover, carvedilol also has antioxidative properties. Carvedilol (1-(4-carbazolyloxy)-3-[2-(2-methoxyphenoxy)ethylamino]-2-propanol) is the object of European Patent No. 0 004 920 and can be manufactured according to the process described there.

In pharmaceutical technology, solid molecular dispersed solutions are a sub-group of solid dispersions. Under a "solid or semi-solid dispersion" there is understood in the pharmaceutical literature the finely dispersed distribution of one or more solids, for example carvedilol and/or a pharmaceutically acceptable salt thereof, in an inert, likewise solid or semi-solid carrier. The active substance can be present in molecular dispersed form, i.e. distributed monomolecularly, as a true solid solution or in fine crystalline dispersed form in a glassy amorphous phase. However, eutectic mixtures, i.e. crystalline structures of active substances and adjuvants, in extremely fine distribution in specific mixture ratios, also fall under this general term. Amongst them, transition forms are possible. The dispersed material starts in size from atoms or molecules and from there can extend to particles measuring several millimeters. Accordingly, an average particle diameter serves as a suitable measurement for the classification of dispersed systems. In general, differentiation is made between molecular dispersed (< 1.0 µm, solid or semi-solid solutions), colloidal dispersed (1-100 µm) and coarsely dispersed (< 0.5 µm) systems. Thereby, it must be taken into consideration that the classification limits have been to some extent established arbitrarily, since the transitions between the individual systems are not clearly defined. True solid solutions are considered in the strict physical sense to be only monophasic systems which result by common crystallization of the components in the form of mixed crystals. Combinations between various possible forms of state frequently result in the production of solid dispersions. The strongest dominating character can be determined by means of X-ray diffraction spectra or differential thermo-analysis.

"Pharmaceutically acceptable salts" of carvedilol embrace alkali metal salts, such as Na or K salts, alkaline earth metal salts, such as Ca and Mg salts, as well as salts with organic or inorganic acids, such as, for example, hydrochloric acid, hydrobromic acid, nitric acid, sulphuric acid, phosphoric acid, citric acid, formic acid, maleic acid, acetic acid, succinic acid, tartaric acid, methanesulphonic acid or toluenesulphonic acid, which are non-toxic for living organisms.

At pH values in the pharmaceutically relevant range of 1 to 8 the solubility of carvedilol in aqueous media lies between about 1 mg and 100 mg per 100 ml (depending on the pH value). This has been found to be problematical especially in the formulations of highly concentrated parenteral formulations, such as e.g. injection solutions or other formulations for the production of small volume administration forms for ocular or oral administration.

In the case of the peroral administration of rapid release carvedilol formulations, e.g. the commercial formulation, resorption quotas of up to 80% are achieved, with a considerable part of the resorbed carvedilol being very rapidly metabolized.

In connection with investigations into the gastrointestinal resorption of carvedilol it has been established that the resorption of carvedilol becomes poorer during the course of passage through the gastrointestinal tract and e.g. in the ileum and colon makes up only a fraction of the resorption in the stomach. This has been found to be very troublesome especially in the development of retard forms in which a release should take place over several hours. The poorer resorption is presumably due entirely or at least in part to the decreasing solubility of carvedilol with increasing pH values. A very low solubility can also be established in the strongly acidic region (about pH 1-2).

In order to improve the resorption quota, especially in the lower regions of the intestine, investigations have been carried out for adjuvants and, respectively, formulations which are suitable for increasing the solubility and/or speed of dissolution of carvedilol.

Accordingly, the underlying purpose of the invention lay in improving the resorption of carvedilol, especially in the case of peroral administration and here especially in the lower regions of the intestine, using agents available in pharmaceutical technology.

Starting from the fact that on the one hand the pH-dependent solubility and on the other hand the speed of dissolution of carvedilol crystals represent the or at least one limiting factor for the resorption of carvedilol, the administration of carvedilol in dissolved form ought to lead to an improved resorption. Since, however, as already described above, the solubility of carvedilol in aqueous media in the pharmaceutically relevant range is very low, the use of a finished medicament in the form of an aqueous solution is excluded for practical reasons.

Attempts have been made especially to provide concentrated, solid peroral formulations in which the active substance is present distributed as a molecular dispersion and accordingly can be resorbed more rapidly.

Some examples of such "solid" molecular dispersed solutions of difficultly soluble medicaments, so-called "solid solutions", are known from the literature. Thus, e.g., a clearly super-saturated solution can be produced transiently by the production of co-precipitates of corticosteroids and polyvinylpyrrolidone (PVP) from organic solvents.

Investigations have established that carvedilol can be dissolved in solutions of polyvinylpyrrolidone (PVP) or hydroxypropylmethylcellulose (HPMC) in organic solvents such as e.g. methylene chloride. After removal of the solvent there are thus obtained solid solutions of carvedilol in PVP or, respectively, HMPC. Polyvinylpyrrolidone which is not cross-linked and which has a molecular weight of 8,000 to 630,000, preferably 25,000, can be used in the formulations.

For industrial applications there are, however, preferred those pharmaceutically acceptable formulations which are produced while avoiding the use of organic solvents:

As an alternative to the aforementioned co-precipitates there also come into consideration solid solutions in the form of so-called "solidified melts". Experiments with several adjuvants, which come into consideration as a basis for such melts, showed, however, that with the "embedding" of carvedilol in these adjuvants either no amorphous state, i.e. no distribution as a molecular dispersion after solidification of the solution, was obtained, that a wholly or partly achieved amorphous state could be maintained only for a short time or that a sufficiently rapid solidification of the melt no longer prevailed.

Surprisingly, it has now been found that carvedilol can be dissolved in certain selected adjuvants under specific conditions, with the distribution of the active substance as a molecular distribution being maintained even at room temperature. Thereby, there are obtained solid or wax-like formulations - so-called solid solutions - in which carvedilol is present in molecular dispersed, i.e. in amorphous, form.

As examples of these adjuvants there are to be named especially adjuvants which are not surface-active, such as polyethylene glycols (PEG) or sugar substitutes as well as non-ionic tensides, such as polyoxyethylene stearates, e.g. Myrj^{®} 52, or polyoxyethylene-polyoxypropylene copolymers, e.g. Pluronic^{®} F 68.

The content of hydrophilic polyoxyethylene groups in the aforementioned polyoxyethylene-polyoxypropylene copolymers preferably lies at 70% to 90%. In an, especially preferred embodiment the ratio of hydrophilic polyoxyethylene groups to hydrophobic polyoxypropylene groups lies at about 80:20 and the average molecular weight preferably lies at about 8,750.

The aforementioned polyoxyethylene stearates preferably have a hydrophilic-lipophilic balance (HLB) value of 10 to 20, preferably of 14 to 20, especially of 16 to 18.

From the series of sugar substitutes especially isomalt (hydrogenated isomaltulose), e.g. Palatine^{®}, has been found to be particularly suitable. Palatinit^{®} is a hydrogenated isomaltulose, which consists of about equal parts of 1-O-α-D-glucopyranosyl-D-sorbitol and 1-0-α-D-glucopyranosyl-D-mannitol dihydrate.

Further, in connection with the present invention polyethylene glycols with a molecular weight of 1,000 to 20,000, preferably 4,000 to 10,000, particularly 6,000 to 8,000, have been found to be especially suitable.

In a preferred embodiment of the present invention the carvedilol is dissolved in a non-ionic tenside, preferably Pluronic^{®} F 68, or in an adjuvant which is not surface-active, preferably polyethylene glycol 6,000.

Thus, carvedilol can be dissolved in polyethylene glycol 6,000 which is melted at about 70°C. In this manner there are obtained highly concentrated solutions of carvedilol (up to 500 mg/ml), with the carvedilol being present distributed as a molecular dispersion in the solution. Moreover, further additives, for example cellulose derivatives such as hydroxypropylmethylcelluloses or hydroxypropylcelluloses, can be admixed in order to control the release rate of the active substance. Further, the compositions in accordance with the invention can contain highly dispersed silicon dioxide as an anti-caking agent.

Concentrated pharmaceutically acceptable solid solutions in which the carvedilol is present distributed as a molecular dispersion can be produced with the aforementioned adjuvants.

The present invention is accordingly concerned with pharmaceutically acceptable compositions comprising carvedilol or a pharmaceutically acceptable salt thereof distributed as a molecular dispersion in a concentration above 5% (wt./wt.).

Under a distribution as a molecular dispersion there is to be understood a monomolecular distribution of the active substance in a suitable carrier.

In a preferred embodiment variant the carvedilol content in the compositions in accordance with the invention lies at 5% (wt./wt.) to 60% (wt./wt.), preferably at 5% (wt./wt.) to 50% (wt./wt.), especially at 10% (wt./wt.) to 40% (wt./wt.), with the weight % details relating to the total weight of the composition (active substance and adjuvant).

Carvedilol formulations which contain such solid solutions in accordance with the invention have a better active substance resorption and thus an improved bioavailability compared with formulations which contain crystalline carvedilol, since the active substance is resorbed more rapidly in dissolved form than from the crystalline state.

The distribution of the carvedilol as a molecular distribution in the base, i.e. the so-called amorphous state (in contrast to the usual crystalline state), can be detected and, respectively, controlled e.g. by means of X-ray diffractometry and/or differential scanning calorimetry (DSC).

Solutions which are solid at room temperature are especially preferred. In a preferred embodiment the adjuvants in accordance with the invention have a melting point below 120°C, especially a melting point of 30°C to 80°C.

The aforementioned adjuvants can be used individually or in a combination of two or more adjuvants with one another. The combination of an adjuvant which is not surface-active, preferably polyethylene glycol, with a non-ionic tenside, preferably a polyoxyethylene-polyoxypropylene copolymer, e.g. Pluronic^{®} F 68, is especially preferred. With these adjuvant mixtures there can on the one hand be produced stable solid solutions of carvedilol and on the other hand the addition of surface-active substances can accelerate the active substance release from the solid solutions.

Solid solutions of carvedilol which contain as adjuvants polyethylene glycol, preferably polyethylene glycol 6,000, as well as 0.1% to 50%, preferably 0.1% to 10%, of polyoxyethylene-polyoxypropylene copolymers, e.g. Pluronic^{®} F 68, have been found to be especially suitable.

In a particular embodiment of the present invention the ratio of the aforementioned adjuvant which is not surface-active, for example polyethylene 6,000 to the surface-active adjuvant, for example Pluronic^{®} F 68, lies between 1000:1 and 1:1, preferably between 100:1 and 10:1.

The solid solutions of carvedilol in accordance with the invention and medicaments produced therefrom can contain further additives such as, for example, binders, plasticizers, diluents, carrier substances, glidants, antistatics, antioxidants, adsorption agents, separation agents, dispersants, dragéeing laquer, de-foamers, film formers, emulsifiers, extenders and fillers.

The aforementioned additives can be organic or inorganic substances, e.g. water, sugar, salts, acids, bases, alcohols, organic polymeric compounds and the like. Preferred additives are lactose, saccharose, tablettose, sodium carboxymethylstarch, magnesium stearate, various celluloses and substituted celluloses such as, for example, methylhydroxy-propylcellulose, polymeric cellulose compounds, highly dispersed silicon dioxide, maize starch, talcum, various polymeric polyvinylpyrrolidone compounds as well as polyvinyl alcohols and their derivatives. It is a prerequisite that all additives used in the production are non-toxic and advantageously do not change the bioavailability of the active substance

In a preferred embodiment the compositions in accordance with the invention contain carvedilol, polyethylene glycol, polyoxyethylene-polyoxypropylene copolymer as well as highly dispersed silicon dioxide. In an especially preferred embodiment the compositions in accordance with the invention contain 10-20% (wt./wt.) carvedilol, 65-85% (wt./wt.) polyethylene glycol, 1-10% (wt./wt.) polyoxyethylene-polyoxypropylene copolymer and 0.1-10% (wt./wt.) highly dispersed silicon dioxide, with the percentages relating to the total weight of the four named substances irrespective of whether additional adjuvants are present in the composition.

When the melt of carvedilol in the aforementioned adjuvants is left to solidify at room temperature, then any crystalline component present in the melt can lead to an acceleration of the crystallisation-out of the amorphous carvedilol.

Surprisingly, it has now been found that an as rapid as possible solidification of the melt of the adjuvant with the dissolved active substance - preferably by spray solidification - leads to particularly stable solid solutions. Altogether, the rapid "freezing up" of the molecular dispersed state of distribution of the carvedilol appears especially to facilitate the maintenance of the amorphous state. This applies e.g. also for the production of solid solutions from solutions which in addition to carvedilol also contain cellulose derivatives, especially hydroxypropylmethylcelluloses or hydroxypropylcelluloses, as a base for "solid solutions", when the solid solution has been produced by means of spray drying. The same also applies for the spray drying of carvedilol and polyvinylpyrrolidone (PVP) from solvents.

In the case of spray drying, the material to be dried is sprayed as a solution or suspension at the upper end of a wide, cylindrical container through an atomizer arrangement to give a droplet mist. The resulting droplet mist is mixed with hot air (preferably > 100°C) or an inert gas which is conducted into the dryer around the atomization zone. The resulting solvent vapour is taken up by the drying air and transported away, and the separated powder is removed from the container via a separator.

In the case of spray solidification, the material to be solidified is sprayed as a melt at the upper end of a wide, cylindrical container through a heatable atomizer arrangement to give a droplet mist. The resulting droplet mist is mixed with cooled air (preferably < 25°C), which is conducted into the dryer around the atomization zone. The heat of solidification which is liberated is taken up by the air and transported away, and the separated solidified powder is removed from the container via a separator. As atomizer arrangements there come into consideration (heatable) rotary pressure nozzles, pneumatic nozzles (binary/ternary nozzles) or centrifugal atomizers.

The solid solutions of carvedilol can be advantageously used pharmaceutically in various ways. Thus, for example, such embedded carvedilol distributed as a molecular dispersion can be processed further to rapid release administration forms, such as, for example, tablets, film tablets, capsules, granulates, pellets, etc. with an improved resorption quotient. This permits under certain circumstances a dosage reduction in comparison with conventional rapid release peroral medicaments which have been produced using crystalline carvedilol.

Carvedilol solid solutions can also be used especially advantageously for the production of medicaments with a modified release characteristic. Under a modified release characteristic there is to be understood, for example, a 95% release after more than two hours, preferably after 2 to 24 hours, or a pH-dependent release in which the beginning of the release is delayed in time. For this purpose, the carvedilol solid solutions can be processed to or with all conventional pharmaceutical oral medicaments with modified release.

Examples of medicaments with a modified release characteristic are film tablets which are resistant to gastric juice or retard forms, such as e.g. hydrocolloid matrices or similar medicaments from which the active substance is released via an erosion or diffusion process. The formulations in accordance with the invention can be processed to formulations with modified active substance release by the addition of further adjuvants or film coatings or by incorporation in conventional pharmaceutical release systems. Thus, the formulations in accordance with the invention can be incorporated, for example, in hydrocolloid matrix systems, especially in those which are based on cellulose derivatives such as hydroxypropylcellulose, hydroxypropylmethylcellulose, methylcellulose or polyacrylate derivatives such as, for example, Eudragit RL. The aforementioned matrices can contain, additionally or alternatively, a hydrocolloid matrix former which swells depending on pH, such as, for example, sodium alginate or sodium carboxymethylcellulose: By the addition of such an adjuvant a targeted release which is individually determined can be achieved. Thereby, the use of the solid solutions in accordance with the invention leads to an appreciable improvement in the resorption in comparison to the crystalline active substance.

Thus, the spray solidified solid solutions of carvedilol in accordance with the invention, preferably those comprising Pluronic^{®} F 68, polyethylene glycol 6000, highly dispersed silicon dioxide and carvedilol (preferably in accordance with Example 4), can be pressed to tablets, for example, by direct compression, granulation and compacting together with hydrophilic matrix formers which control the release, such as e.g. hydroxypropylmethylcelluloses 2208 with an average viscosity of about 100 mPa•s (Methocel^{®} K100 LV-Premium) and hydroxypropylmethylcelluloses 2208 with an average viscosity of about 4000 mPa · s (Methocel^{®} K4M-Premium), and with glidants or anti-caking agents, such as e.g. magnesium stearate and microcrystalline celluloses (Avicel^{®} PH102). Moreover, the tablets can be coated with a conventional lacquer, such as e.g. Opadryl^{®} II White Y-30-18037 and Opadryl^{®} Clear YS-1-7006.

The pharmaceutical formulations in accordance with the invention are suitable for the production of conventional pharmaceutical administration forms, preferably oral administration forms, for the treatment and/or prophylaxis of cardiac and circulatory disorders, such as e.g. hypertension, cardiac insufficiency and angina pectoris.

The dosage in which the pharmaceutical formulations in accordance with the invention are administered depends on the age and the requirements of the patients and the route of administration. In general, dosages of about 1 mg to 50 mg of carvedilol,per day come into consideration. For this, formulations with a carvedilol active substance content of about 1 mg to 50 mg are used.

The present invention is also concerned with a process for the production of concentrated solid or semi-solid molecular dispersed solutions of carvedilol, which comprises the admixture of carvedilol with hydrophilic adjuvants, such as, for example, polyethylene glycol, and/or surface-active substances, such as, for example, Pluronic^{®}F 68. In a preferred embodiment the thus-obtained formulation is subsequently spray solidified.

Further, the present invention is concerned with the use of said formulations for the manufacture of a medicament for the treatment of illnesses, such as hypertension, cardiac insufficiency or angina pectoris, which comprises the administration of medicaments which contain the pharmaceutical formulations described above.

The following Examples are intended to describe the preferred embodiments of the present invention, without thereupon limiting this.

### Example 1

Carvedilol solid solution:

| | |
|---|---|
| Carvedilol | 50.0 g |
| Polyethylene glycol 6,000 | 250.0 g |
| | |
| Total weight: | 300.0 g |

The polyethylene glycol 6,000 is melted at 70°C. The carvedilol is stirred into the resulting melt and homogeneously dissolved. Then, the melt is spray solidified to the carvedilol solid solution. Alternatively, the melt can be solidified by means of other methods, provided that the solidification takes place rapidly.

### Example 2

Carvedilol solid solution:

| | |
|---|---|
| Carvedilol | 50.0 g |
| Polyoxyethylene-polyoxypropylene copolymer | 250.0 g |
| | |
| Total weight: | 300.0 g |

The polyoxyethylene-polyoxypropylene copolymer is melted at 70°C. The carvedilol is stirred into the resulting melt and homogeneously dissolved. Then, the melt is spray solidified to the carvedilol solid solution. Alternatively, the melt can be solidified by means of other methods, provided that the solidification takes place rapidly.

### Example 3

Carvedilol solid solution:

| | |
|---|---|
| Carvedilol | 50.0 g |
| Polyoxyethylene-polyoxypropylene copolymer | 15.0 g |
| Polyethylene glycol 6,000 | 235.0 g |
| | |
| Total weight: | 300.0 g |

The polyethylene glycol 6,000 is melted at 70°C. Subsequently, the polyoxyethlene-polyoxypropylene copolymer is stirred into the above melt, likewise melted and the melt is homogenized. The carvedilol is stirred into the resulting melt and homogeneously dissolved. Then, the melt is spray solidified to the carvedilol solid solution. Alternatively, the melt can be solidified by means of other methods, provided that the solidification takes place rapidly.

If desired, the technical processing properties such as, for example, the flowability of the solid solutions can be improved by the addition of further adjuvants, see Example 4.

### Examples

Carvedilol solid solution:

| | |
|---|---|
| Carvedilol | 50.0 g |
| Polyoxyethylene-polyoxypropylene copolymer | 15.0 g |
| Polyethylene glycol 6,000 | 232.0 g |
| Silicon dioxide, highly dispersed | 3.0 g |
| | |
| Total weight: | 300.0 g |

The polyethylene glycol 6,000 is melted at 70°C. Subsequently, the polyoxyethlene-polyoxypropylene copolymer is stirred into the above melt, likewise melted and the melt is homogenized. The carvedilol is stirred into the resulting melt and homogeneously dissolved. Then, the melt is spray solidified to the carvedilol solid solution. Alternatively, the melt can be solidified by means of other methods, provided that the solidification takes place rapidly. The carvedilol solid solution is treated with highly dispersed silicon dioxide and mixed homogeneously.

Also, higher content of surface-active adjuvant give stable amorphous embeddings.

### Example 5

Carvedilol solid solution:

| | |
|---|---|
| Carvedilol | 50.0 g |
| Polyoxyethylene-polyoxypropylene copolymer | 125.0 g |
| Polyethylene glycol 6,000 | 125.0 g |
| | |
| Total weight: | 300.0 g |

The polyethylene glycol 6,000 is melted at 70°C. Subsequently, the polyoxyethlene-polyoxypropylene copolymer is stirred into the above melt, likewise melted and the melt is homogenized. The carvedilol is stirred into the resulting melt and homogeneously dissolved. Then, the melt is spray solidified to the carvedilol solid solution. Alternatively, the melt can be solidified by means of other methods, provided that the solidification takes place rapidly.

### Example 6

Carvedilol solid solution:

| | |
|---|---|
| Carvedilol | 50.0 g |
| Isomalt | 450.0 g |
| | |
| Total weight: | 500.0 g |

The isomalt is melted at above its melting point. Subsequently, the carvedilol is stirred into the resulting melt and homogeneously dissolved. Then, the melt is spray solidified to the carvedilol solid solution. Alternatively, the melt can be solidified by means of other methods, provided that the solidification takes place rapidly.

### Example 7

Rapid release carvedilol tablets using a solid solution:

| | |
|---|---|
| Carvedilol | 50.0 g |
| Polyoxyethylene-polyoxypropylene copolymer | 15.0 g |
| Polyethylene glycol 6,000 | 232.0 g |
| Silicon dioxide, highly dispersed | 3.0 g |
| Tablettose | 146.0 g |
| Sodium carboxymethylstarch | 15.0 g |
| Silicon dioxide, highly dispersed | 4.0 g |
| Magnesium stearate | 10.0 g |
| | |
| Total weight: | 475.0 g |

The polyethylene glycol 6,000 is melted at 70°C. Subsequently, the polyoxyethlene-polyoxypropylene copolymer is stirred into the above melt, likewise melted and the melt is homogenized. The carvedilol is stirred into the resulting melt and homogeneously dissolved. Then, the melt is spray solidified to the carvedilol solid solution. Alternatively, the melt can be solidified by means of other methods, provided that the solidification takes place rapidly. The carvedilol solid solution is subsequently treated with highly dispersed silicon dioxide and mixed homogeneously. The mixture obtained is treated with tablettose and mixed. The outer phase (lubricant, flow agent, separating agent and extender) consisting of sodium carboxymethylstarch, highly dispersed silicon dioxide and magnesium stearate is added to the above mixture and mixed homogeneously. The resulting mixture is then pressed to pharmaceutical forms or filled into capsules in the usual manner taking into consideration the desired active substance content.

### Example 8

Carvedilol retard tablets:

| | |
|---|---|
| Carvedilol | 50.0 g |
| Polyoxyethylene-polyoxypropylene copolymer | 15.0 g |
| Polyethylene glycol 6,000 | 232.0 g |
| Silicon dioxide, highly dispersed | 3.0 g |
| Tablettose | 146.0 g |
| Hydroxypropylmethylcellulose 2208 | 240.0 g |
| Silicon dioxide, highly dispersed | 4.0 g |
| Magnesium stearate | 10.0 g |
| | |
| Total weight: | 700.0 g |

The polyethylene glycol 6,000 is melted at 70°C. Subsequently, the polyoxyethlene-polyoxypropylene copolymer is stirred into the above melt, likewise melted and the melt is homogenized. The carvedilol is stirred into the resulting melt and homogeneously dissolved. Then, the melt is spray solidified to the carvedilol solid solution. Alternatively, the melt can be solidified by means of other methods, provided that the solidification takes place rapidly. The carvedilol solid solution is subsequently treated with highly dispersed silicon dioxide and mixed homogeneously. The mixture obtained is treated with tablettose and mixed. The outer phase (lubricant, flow agent, separating agent and extender), consisting of hydroxypropylmethylcellulose 2208, highly dispersed silicon dioxide and magnesium stearate is added to the above mixture and mixed homogeneously. The resulting mixture is then pressed to pharmaceutical forms or filled into capsules in the usual manner taking into consideration the desired active substance content.

### Example 9

Carvedilol retard tablets:

| | |
|---|---|
| Carvedilol | 50.0 g |
| Polyoxyethylene-polyoxypropylene copolymer | 15.0 g |
| Polyethylene glycol 6,000 | 232.0 g |
| Silicon dioxide, highly dispersed | 3.0 g |
| Tablettose | 96.0 g |
| Hydroxypropylmethylcellulose 2208 | 240.0 g |
| Sodium alginate | 50.0 g |
| Silicon dioxide, highly dispersed | 4.0 g |
| Magnesium stearate | 10.0 g |
| | |
| Total weight: | 700.0 g |

The polyethylene glycol 6,000 is melted at 70°C. Subsequently, the polyoxyethlene-polyoxypropylene copolymer is stirred into the above melt, likewise melted and the melt is homogenized. The carvedilol is stirred into the resulting melt and homogeneously dissolved. Then, the melt is spray solidified to the carvedilol solid solution. Alternatively, the melt can be solidified by means of other methods, provided that the solidification takes place rapidly. The carvedilol solid solution is subsequently treated with highly dispersed silicon dioxide and mixed homogeneously. The mixture obtained is treated with tablettose and mixed. The outer phase (lubricant, flow agent, separating agent and extender), consisting of sodium alginate, highly dispersed silicon dioxide and magnesium stearate is added to the above mixture and mixed homogeneously. The resulting mixture is then pressed to pharmaceutical forms or filled into capsules in the usual manner taking into consideration the desired active substance content.

## Claims

1. A pharmaceutically acceptable composition comprising carvedilol or a pharmaceutically acceptable salt thereof distributed as a molecular dispersion in a concentration above 5% wt./wt..

2. The composition according to claim 1, which is a solid or semi-solid solution.

3. The compositions according to any one of claims 1 to 2, wherein one or more adjuvants which are not surface-active are present.

4. The composition according to claim 3, wherein polyethylene glycol is present as the adjuvant which is not surface-active.

5. The composition according to claim 4, wherein the polyethylene glycol has a molecular weight of 1,000 to 20,000, preferably 4,000 to 10,000.

6. The composition according to any one of claims 1 to 5, wherein a sugar substitute is present as the adjuvant which is not surface-active,

7. The composition according to claim 6, wherein isomalt is present as the sugar substitute.

8. The composition according to any one of claims 1 to 7, wherein one or more non-ionic tensides are present.

9. The composition according to claim 8, wherein the solution contains a polyoxyethylene-polyoxypropylene copolymer as the non-ionic tenside.

10. The composition according to any one of claims 7 to 8, wherein the solution contains a polyoxyethylene stearate as the non-ionic tenside.

11. The composition according to any one of claims 7 to 9, wherein the ratio of adjuvants which are not surface active to non-ionic tensides lies between 1000:1 and 1:1, preferably between 100:1 and 10:1.

12. The composition according to any one of claims 1 to 11, wherein the carvedilol concentration lies between 5% wt./wt. and 60% wt./wt..

13. The composition according to any one of claims 1 to 11, wherein the carvedilol concentration lies between 10% wt./wt. and 40% wt./wt..

14. The composition according to any one of claims 1 to 13, wherein highly dispersed silicon dioxide is present.

15. A composition according to any one of claims 1 to 14, which contains 10-20% wt./wt. carvedilol, 65-85% wt./wt. polyethylene glycol, 1-10% wt./wt. polyoxyethylene-polyoxypropylene copolymer and 0.1-10% wt./wt highly dispersed silicon dioxide.

16. A pharmaceutically acceptable administration form comprising a composition according to any one of claims 1 to 15.

17. The pharmaceutically acceptable administration form according to claim 16, which has a modified active substance release, with 95% of the active substance being released in 2 to 24 hours.

18. The pharmaceutically acceptable administration form according to claim 16 or claim 17, which is a solid administration form.

19. The pharmaceutically acceptable administration form according to claim 16 or claim 17, which is an oral administration form.

20. A process for the production of a composition, according to any one of claims 1 to 15, which process comprises mixing the carvedilol with an adjuvant which is not surface-active and/or a non-ionic tenside.

21. The process according to claim; 20, wherein the resulting melt is solidified by spray solidification.

22. The use of a composition according to any one of claims 1 to 15 for the production of medicaments for the treatment or prophylaxis of illnesses such as hypertension, cardiac insufficiency or angina pectoris.

## Patentansprüche

1. Pharmazeutisch verträgliche Zusammensetzung umfassend Carvedilol oder ein pharmazeutisch verträgliches Salz davon, welche als eine molekulare Dispersion in einer Konzentration von über 5% Gew./Gew. verteilt ist.

2. Zusammensetzung gemäß Anspruch 1, welche ein Feststoff oder eine halbfeste Lösung ist.

3. Zusammensetzung gemäß einem der Ansprüche 1 bis 2, wobei ein oder mehrere nicht oberflächenaktive Hilfsstoffe vorhanden sind.

4. Zusammensetzung gemäß Anspruch 3, wobei Polyethylenglycol als nicht oberflächenaktiver Hilfsstoff vorhanden ist.

5. Zusammensetzung gemäß Anspruch 4, wobei das Polyethylenglycol ein Molekulargewicht von 1.000 bis 20.000, bevorzugt 4.000 bis 10.000, aufweist.

6. Zusammensetzung gemäß einem der Ansprüche 1 bis 5, wobei ein Zuckerersatzstoff als nicht oberflächenaktiver Hilfsstoff vorhanden ist.

7. Zusammensetzung gemäß Anspruch 6, wobei Isomalt als Zuckerersatzstoff vorhanden ist.

8. Zusammensetzung gemäß einem der Ansprüche 1 bis 7, wobei ein oder mehrere nichtionische Tenside vorhanden sind.

9. Zusammensetzung gemäß Anspruch 8, wobei die Lösung ein Polyoxyethylen-Polyoxypropylen-Copolymer als nichtionisches Tensid enthält.

10. Zusammensetzung gemäß einem der Ansprüche 7 bis 8, wobei die Lösung ein Polyoxyethylenstearat als nichtionisches Tensid enthält.

11. Zusammensetzung gemäß einem der Ansprüche 7 bis 9, wobei das Verhältnis der nicht oberflächenaktiven Hilfsstoffe zu den nichtionischen Tensiden zwischen 1000:1 und 1:1, bevorzugt zwischen 100:1 und 10:1, liegt.

12. Zusammensetzung gemäß einem der Ansprüche 1 bis 11, wobei die Carvedilolkonzentration zwischen 5% Gew./Gew. und 60% Gew./Gew.liegt.

13. Zusammensetzung gemäß einem der Ansprüche 1 bis 11, wobei die Carvedilolkonzentration zwischen 10% Gew./Gew. und 40% Gew./Gew. liegt.

14. Zusammensetzung gemäß einem der Ansprüche 1 bis 13, wobei hochdispergiertes Siliciumdioxid vorhanden ist.

15. Zusammensetzung gemäß einem der Ansprüche 1 bis 14, welche 10 bis 20% Gew./Gew. Carvedilol, 65 bis 85% Gew./Gew. Polyethylenglycol, 1 bis 10% Gew./Gew. Polyoxyethylen-Polyoxypropylen-Copolymer und 0,1 bis 10% Gew./Gew. hochdispergiertes Siliciumdioxid enthält.

16. Pharmazeutisch verträgliche Verabreichungsform, umfassend eine Zusammensetzung gemäß einem der Ansprüche 1 bis 15.

17. Pharmazeutisch verträgliche Verabreichungsform gemäß Anspruch 16, welche eine modifizierte Wirkstofffreisetzung aufweist, wobei 95% des Wirkstoffs in 2 bis 24 Stunden freigesetzt werden.

18. Pharmazeutisch verträgliche Verabreichungsform gemäß Anspruch 16 oder Anspruch 17, welche eine feste Verabreichungsform ist.

19. Pharmazeutisch verträgliche Verabreichungsform gemäß Anspruch 16 oder Anspruch 17, welche eine orale Verabreichungsform ist.

20. Verfahren zur Herstellung einer Zusammensetzung gemäß einem der Ansprüche 1 bis 15, wobei das Verfahren das Mischen des Carvedilols mit einem nicht oberflächenaktiven Hilfsstoff und/oder einem nichtionischen Tensid umfasst.

21. Verfahren gemäß Anspruch 20, wobei die erhaltene Schmelze durch Sprayhärtung gehärtet wird.

22. Verwendung einer Zusammensetzung gemäß einem der Ansprüche 1 bis 15 zur Herstellung von Medikamenten zur Behandlung oder Prophylaxe von Erkrankungen wie Hypertonie, Herzinsuffizienz oder Angina pectoris.

## Revendications

1. Composition pharmaceutiquement acceptable comprenant du carvédilol ou un de ses sels pharmaceutiquement acceptables, réparti en une dispersion moléculaire à une concentration supérieure à 5 % en poids/poids.

2. Composition suivant la revendication 1, qui est une solution solide ou semi-solide.

3. Composition suivant l'une quelconque des revendications 1 et 2, dans laquelle un ou plusieurs adjuvants qui sont non tensioactifs sont présents.

4. Composition suivant la revendication 3, dans laquelle du polyéthylèneglycol est présent comme adjuvant qui est non tensioactif.

5. Composition suivant la revendication 4, dans laquelle le polyéthylèneglycol a un poids moléculaire de 1000 à 20 000, de préférence de 4000 à 10 000.

6. Composition suivant l'une quelconque des revendications 1 à 5, dans laquelle un produit de substitution de sucre est présent comme adjuvant qui est non tensioactif.

7. Composition suivant la revendication 6, dans laquelle de l'isomalt est présent comme produit de substitution de sucre.

8. Composition suivant l'une quelconque des revendications 1 à 7, dans laquelle un ou plusieurs agents tensioactifs non ioniques sont présents.

9. Composition suivant la revendication 8, dans laquelle la solution contient un copolymère polyoxyéthylène-polyoxypropylène comme agent tensioactif non ionique.

10. Composition suivant l'une quelconque des revendications 7 et 8, dans laquelle la solution contient un poly(stéarate d'oxyéthylène) comme agent tensioactif non ionique.

11. Composition suivant l'une quelconque des revendications 7 à 9, dans laquelle le rapport des adjuvants qui sont non tensioactifs aux agents tensioactifs non ioniques est de 1000:1 à 1:1, de préférence de 100:1 à 10:1.

12. Composition suivant l'une quelconque des revendications 1 à 11, dans laquelle la concentration en carvédilol est de 5 % en poids/poids à 60 % en poids/poids.

13. Composition suivant l'une quelconque des revendications 1 à 11, dans laquelle la concentration en carvédilol est de 10 % en poids/poids à 40 % en poids/poids.

14. Composition suivant l'une quelconque des revendications 1 à 13, dans laquelle du dioxyde de silicium hautement dispersé est présent.

15. Composition suivant l'une quelconque des revendications 1 à 14, qui contient 10 à 20 % en poids/poids de carvédilol, 65 à 85 % en poids/poids de polyéthylèneglycol, 1 à 10 % en poids/poids de copolymère polyoxyéthylène-polyoxypropylène et 0,1 à 10 % en poids/poids de dioxyde de silicium hautement dispersé.

16. Forme d'administration pharmaceutiquement acceptable comprenant une composition suivant l'une quelconque des revendications 1 à 15.

17. Forme d'administration pharmaceutiquement acceptable suivant la revendication 16, qui présente une libération de substance active modifiée, une proportion de 95 % de la substance active étant libérée en 2 à 24 heures.

18. Forme d'administration pharmaceutiquement acceptable suivant la revendication 16 ou la revendication 17, qui est une forme d'administration solide.

19. Forme d'administration pharmaceutiquement acceptable suivant la revendication 16 ou la revendication 17, qui est une forme d'administration orale.

20. Procédé pour la production d'une composition suivant l'une quelconque des revendications 1 à 15, procédé qui comprend le mélange du carvédilol à un adjuvant qui est non tensioactif et/ou un agent tensioactif non ionique.

21. Procédé suivant la revendication 20, dans lequel la masse fondue résultante est solidifiée par solidification par atomisation.

22. Utilisation d'une composition suivant l'une quelconque des revendications 1 à 15, pour la production de médicaments destinés au traitement ou à la prophylaxie de maladies telles que l'hypertension, l'insuffisance cardiaque ou l'angine de poitrine.
